# EUROPEAN PATENT APPLICATION

(11) **EP 2 407 194 A1**
(43) Date of publication of application: **18.01.2012**
(21) Application number: 10305774.1
(22) Date of filing: 12.07.2010
(51) Int. Cl.: A61M 5/24, A61L 29/16, A61M 5/32

(54) **Injection system for mixing two injectable compositions prior to injection**

(71) Applicant: SOFIC (Sté Française d'Instruments de Chirurgie), 81200 Mazamet (FR)
(72) Inventor: Lambert, Grégory, 92290, Châtenay-Malabry (FR); Wollbold, Jürgen, 81290, Labruguière (FR); Combes, Christophe, 81200, Mazamet (FR)
(74) Representative: Icosa

(57) **Abstract**

This invention relates to a method for mixing a first injectable liquid pharmaceutical composition and a second injectable composition within an injection device, said injection device including:
• a generally cylindrical barrel or a generally cylindrical shell in which a carpule is lodged,
• at one first end of the generally cylindrical barrel or the generally cylindrical shell, a plunger, the plunger being mounted on the barrel or shell,
• at a second end opposite the first end of the generally cylindrical barrel or the generally cylindrical shell, is provided a needle assembly support member, on which may be mounted a needle assembly, said needle assembly comprising means to be mounted on the needle support member, and further comprising:
• at least one hollow canula for transportation of liquid from the barrel or carpule to the body of the patient;
• and at least one hub surrounding part of the canula and attached to canula; said hub possibly including a hollow part;

characterized in that the first injectable composition is within the cylindrical barrel or the carpule and the second injectable composition is associated with the needle assembly, and further characterized in that the mixture of said first and said second composition occurs in the hub, in the carpule, or in the canula. The invention also relates to said device.

## Description

### [Field of invention]

This invention relates to the field of injectable compositions. More specifically, this invention relates to a method for mixing two injectable compositions prior to injection. This invention also relates to an injection device hereinafter called a syringe, comprising two distinct pharmaceutical compositions and means for mixing both compositions prior to injection.

In a particular embodiment, this invention relates to a method and a device for neutralizing and/or buffering at least one injectable composition having a non-physiological pH by contacting it, prior to injection, with a second composition designed for neutralizing and/or buffering the pH of the first composition.

The injection device of the invention may be used, in particular but not exclusively, in dentistry applications The first composition may be, for example, a local anesthetic composition for dentistry purposes and the second composition may be, for example, a buffer suitable for buffering said local anesthetic composition. The invention is especially useful for local anesthesia, but also for loco-regional anesthesia and regional anesthesia as well.

### [Background of the invention]

This issue of mixing two injectable compositions, for various purposes, prior to injection, is well-known in the art.

It is known that the storage abilities of a number of pharmaceutical compositions are directly linked to their acidic or basic pH, and that a buffering process has to be implemented extemporaneously shortly prior to injection.

Particularly, the issue of buffering a composition to be injected, having a non-physiological pH, prior to injection, is a constant issue in the field of dental anesthetics.

For example, US 2009/221,984 describe a method and apparatus for buffering lidocaine (with and without epinephrine) while improving shelf life without the need to refrigerate. Neutralizing the pH of dental anesthetics prior to injection may also improve efficacy of these anesthetics, while lowering the pain of the patient during injection and following minutes.

Nevertheless, the operator/practitioner usually performing the surgical act of injecting these compositions is highly reluctant to any amendment or modification of his/her surgical gesture and to any complicated procedure to be performed extemporaneously. Moreover, the manufacturers of carpules are also reluctant to any modification of their containers and the content thereof, as a mere modification may lead to a loss in stability or liability of the product. There is thus still a need for a very simple method and device for mixing two compositions prior to injection.

This invention thus addresses the issue of mixing, prior to injection, a composition enclosed in a carpule or a barrel. More specifically, this invention addresses the issue of buffering, prior to injection, a composition enclosed in a carpule and having a non-physiological pH, using a regular injection device and, anytime possible, letting the operator keep its usual surgical gesture of injection.

When considering the dental field, the method and device of the invention are especially advantageous in that they are safe and provide an efficient solution for preventing pain occurring when a patient is injected with a dental anesthetic. Especially, when the site of injection is an inflammatory area, where the local pH is lowered than regular body pH due to inflammation, the injection of an acidic composition of anesthetic may be very painful. The present invention brings a solution for pain prevention.

Moreover, some dental anesthetics are activated after injection by elevation of their pH at contact with the tissues and body fluids having a regular pH of about 6.8 to 7.4 : when the tissues are in an inflammatory area and have a lower pH, activation of the dental anesthetics cannot correctly be performed in situ and the success rate of the anesthesia is lowered. This invention provides a solution for enhancing the success rate of anesthesia, even on an inflammatory site.

Most importantly, mixing a buffer with a dental anesthetic may also improve duration of action and/or efficacy of the dental anesthetic.

### [Summary of the invention]

This invention thus relates to a method for mixing a first injectable liquid pharmaceutical composition and a second injectable composition within an injection device, said injection device including:
- a generally cylindrical barrel or a generally cylindrical shell in which a carpule is lodged,
- at one first end of the generally cylindrical barrel or the generally cylindrical shell, a plunger, the plunger being mounted on the barrel or shell
- at a second end opposite the first end of the generally cylindrical barrel or the generally cylindrical shell, is provided a needle assembly support member, on which may be mounted a needle assembly, said needle assembly comprising means to be mounted on the needle support member, and further comprising
   o at least one hollow canula for transportation of liquid from the barrel or carpule to the body of the patient,
   o and at least one hub surrounding part of the canula and attached to canula; said hub possibly including a hollow part;
characterized in that the first injectable composition is within the cylindrical barrel or the carpule and the second injectable composition is associated with the needle assembly, and further characterized in that the mixture of said first and said second composition occurs in the hub, in the carpule, or in the canula.

According to an embodiment, the first composition is an injectable composition having a non-physiological pH and the second composition is a buffer of the first composition. In this embodiment, the method of the invention is a method for buffering a first composition with a second composition. Advantageously, the buffer has a pK of 6.5 to 7.8, preferably 7 to 7.4. Preferably, the buffer is NaHCO₃ or any equivalent buffer known from one skilled in the art, having the capacity of buffering the first composition to a pH of 6.5 to 7.8, preferably 7 to 7.4, preferably those described in the European and/or US Pharmacopeia.

According to another embodiment, the second composition is an activator of the first composition. In this embodiment, the method of the invention is a method for activating a first composition with a second composition.

In a particular aspect of the method, the first composition is a liquid and the second composition is in the form of a solid, preferably a powder. In this embodiment, advantageously, the second composition is highly soluble in the first composition.

In another aspect of the method, the first composition is a liquid and the second composition is also in the form of a liquid, preferably a solution or an emulsion. Advantageously, the two liquid compositions are miscible. Preferably, the two compositions are polar compositions. In a preferred embodiment, the ratio of the volume of the second composition to the volume of the first composition is less than one, preferably less than 0.1, more preferably less than 0.05, even more preferably less than 0.03. In a preferred embodiment, the ratio of the volume of the second composition to the volume of the first composition ranges from 0.01 to 0.03.

According to a first embodiment, the volume of the first composition ranges from 1.7 to 1.8 ml or is of about 2.2 ml, when in liquid form.

According to the invention, the second composition is associated with the needle assembly; preferably, the term "associated with" means that the second composition is located on or in the canula, or on or in the hub. According to a first embodiment, the second composition is on the canula. According to a second embodiment, the second composition is in the canula. According to a third embodiment, the second composition is in the hub. According to a fourth embodiment, the second composition is on the hub. According to an embodiment, the second composition may be coated onto the interior and/or outer walls of the canula(s). In this embodiment, the second composition is preferably in a solid form.

According to another embodiment, the volume of the second composition, preferably a buffer, is of about 50 microliters when in liquid form.

According to an embodiment, the first composition is an anesthetic composition, with or without a vasoconstrictor. The anesthetic may preferably be selected from the group comprising or consisting of lidocaine, mepivacaine, prilocaine and articaine and mixtures thereof. The vasoconstrictor may preferably be selected from the group consisting of epinephrine and levonordefin, and mixtures thereof. Preferably, the first composition is selected from lidocaine with or without epinephrine, mepivacaine with or without levonordefin, prilocaine with or without epinephrine, articaine with or without epinephrine. In a embodiment, the first composition is selected from:
lidocaine HCl injection 2%,
mepivacaine HCl injection USP, Plain, 3%,
Prilocaine HCl injection USP, Plain, 4%,
Articaine HCl With Epinephrine USP, 4% 1:100,000,
Articaine HCl With Epinephrine USP, 4% 1:200,000,
lidocaine With Epinephrine injection USP, 2% 1:50,000 to 1:200,000
Mepivacaine HCl With Levonordefrin Injection USP, 2% 1:20,000
Prilocaine With Epinephrine Injection USP, 4% 1:200,000
Bupivacaine With Epinephrine Injection USP, 0.5% 1:200,000.

This invention also relates to an injection device comprising:
- a generally cylindrical barrel or a generally cylindrical shell in which a carpule is lodged,
- at one first end of the generally cylindrical barrel or the generally cylindrical shell, a plunger, the plunger being mounted on the barrel or shell
- at a second end opposite the first end of the generally cylindrical barrel or the generally cylindrical shell, is provided a needle assembly support member, on which may be mounted a needle assembly, said needle assembly comprising means to be mounted on the needle support member, and further comprising
   o at least one hollow canula for transportation of liquid from the barrel or carpule to the body of the patient,
   o and at least one hub surrounding part of the canula and attached to canula; said hub possibly including a hollow part;
characterized in that the first injectable composition is within the cylindrical barrel or the carpule and the second injectable composition is associated with the needle assembly, and further characterized in that the injection device includes means for mixing the first and the second composition, such that the mixture of said first and said second composition occurs in the hub, in the carpule, or in the canula.

The mixing means may be the plunger, as it has the function of engaging the barrel or carpule and of applying pressure to the first composition to dispense the first composition through the needle assembly. As a matter of example, when the plunger is pushed forwardly, it will apply pressure to the first composition within the carpule or barrel so as to dispense the composition through the needle assembly and contact the second composition stored in the needle assembly, so that both compositions are mixed. After this first action, a further action on the plunger may dispense the mixture outside the device through the canula. The mixing means may then be a shift of the plunger, but also an action leading to a displacement of the carpule, or an action on the needle assembly, or any suitable means for contacting first and second composition. According to a further aspect of the invention, the mixing of first and second composition may result from the displacement, preferably from the first displacement, of the first composition. Consequently, any action or means resulting in the first displacement of the first composition to the needle assembly may be considered as a suitable mixing means.

In an embodiment, the needle assembly support member comprises together a thread arranged to cooperate with the needle assembly, more specifically with a corresponding thread on a hub or the needle assembly. When the needle assembly is cooperating with, i.e. reversibly or irreversibly fixed to, the needle assembly support, one end of the canula is within the carpule or cylindrical barrel, whereas the other end extends outside the hub for use for injection into the patient's tissue.

In another aspect of the invention, the needle assembly may comprise at least one canula, and at least one hub.

According to an embodiment, the device of the invention may include two or three distinct canulas.

In an embodiment, the hub in itself defines a hollow chamber capable of receiving the second composition, either in a liquid or in a solid form. In another embodiment, the hub is mounted on the needle support member and together they define a hollow chamber capable of receiving the second composition, either in a liquid or in a solid form. In an embodiment, the needle assembly comprises two hubs, one hub being mounted on the needle assembly support member of the injection device, and the other one being attached to the canula, both hubs being connected one to another and possibly defining a hollow chamber. In an embodiment, the two hubs are adapted to slide relatively to each other.

According to an embodiment, the device of the invention is a disposable syringe, intended for one single use only, preferably packed in a bag. In this embodiment, preferably, the device of the invention may be sterile. In this embodiment, the device of the invention may include a fixed barrel prefilled with the first composition and a needle assembly prefilled with the second composition.

According to another embodiment, the device of the invention is reusable and may be referred to as a carpule syringe: a carpule syringe facilitates repeated use of the device, specifically repeated loading and discharges of carpules containing a specific dose of first composition. A carpule syringe is characterized by the absence of a fixed barrel in which a piston reciprocates. Instead, a carpule syringe includes a cylindrical shell with an opening designed to receive and secure a prefilled carpule including first composition, the ends of which carpule are closed and hermetically sealed. The device is provided with a reciprocatable plunger arranged to engage with one end of the carpule. According to an embodiment, the carpule is unidose and the needle assembly contains exactly the amount of second composition to be mixed with the unidose carpule.

According to an embodiment, the carpule is a prefilled carpule, adapted to be received in the cylindrical shell of the device.

In a preferred embodiment of the invention, the first and the second composition are sterile. According to an embodiment, the needle assembly, including the second composition, is sterile.

According to an embodiment, the second composition is enclosed in a container surrounding the canula, the container being preferably located in the part of the canula which is inside the carpule or the cylindrical barrel containing the first composition, and the container being capable to release the second composition in the carpule or in the cylindrical barrel.

Other characteristics, objects and advantages of the invention will become apparent from the following detailed description provided with reference to the attached drawings, which represent preferred embodiments, by way of non-limiting examples.

### [Brief description of the drawings]

Figure 1 is a perspective view of the device of the invention.
Figure 2A is a side-cross section view of canula 3, wherein the second composition is in the form of a powder coated inside the canula, i.e. onto the interior walls of the canula.
Figure 2B is a side-cross section view of canula 3, wherein the second composition is in the form of a powder coated outside the canula, i.e. onto the outer walls of the canula.
Figure 2C is a side-cross section view of canula 3, wherein the second composition is inside the canula but not coated on the walls of the canula.
Figure 3A is a side-cross section view of an embodiment of the invention, wherein the device comprises a needle assembly defining a hollow chamber.
Figure 3B is a side-cross section view of an embodiment of the invention, wherein the needle assembly defines a hollow chamber and also comprising two needles.
Figure 3C is a side-cross section view of an embodiment of the invention, wherein the needle assembly defines a hollow chamber and the connection between the carpule and the hollow chamber is a corridor.
Figure 4 is a side-cross section view of a particular embodiment of the invention including a hollow chamber and where the canula 3 has an orifice located within the hollow chamber.
Figure 5 is a side-cross section view of a particular embodiment of the invention where the second composition is released within the carpule or cylindrical barrel.
Figure 6 is a side-cross section view of a particular embodiment of the invention where the device includes two imbricated needles.
Figure 7A to 7D are side-cross section views of a particular embodiment of the invention comprising a hub comprising two parts, and three canulas.

### [Detailed description of the drawings]

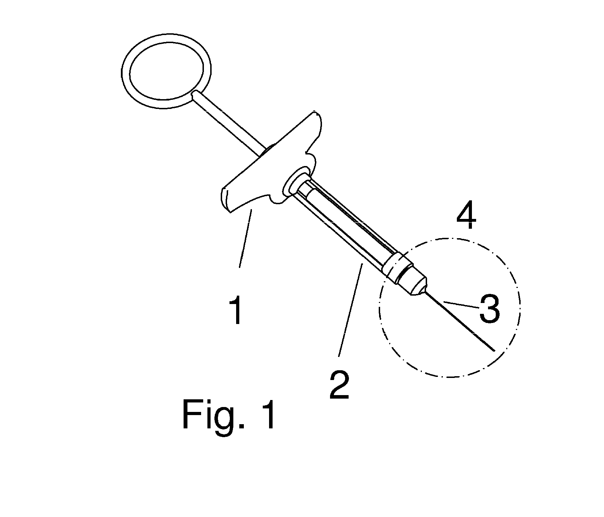

As shown in Fig.1 the device of the invention may be a syringe **1** comprising a cylindrical barrel or a carpule 2 including a first composition and a needle assembly **4,** including a second composition **6,** said needle assembly including at least one canula **3** and at least one hub **7.**

According to an embodiment of the invention, the second composition **6** may be associated with the canula **3,** which means that it is within canula **3** and/or on the outer walls of the canula **3.**

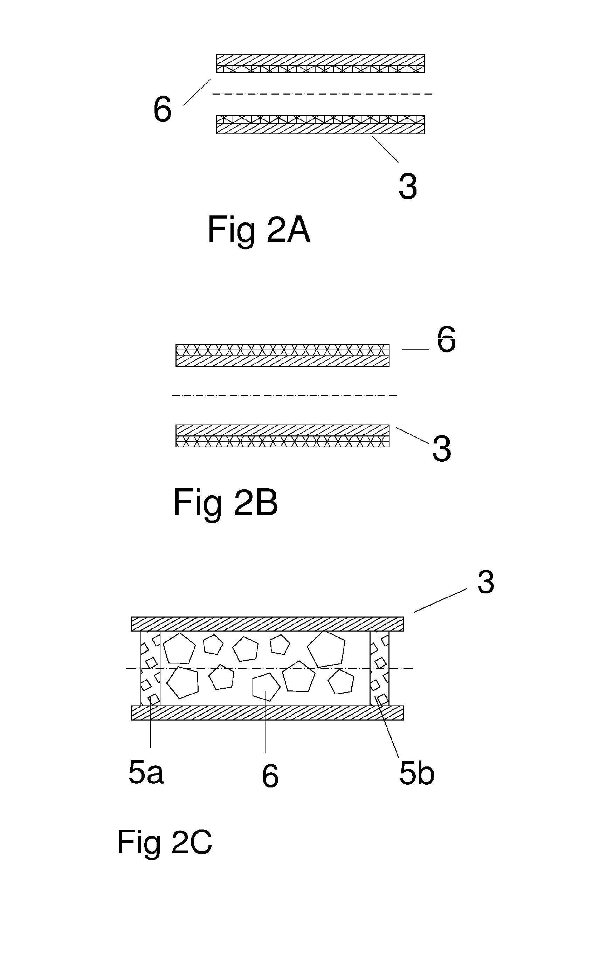

In a first embodiment, the second composition **6** is in a solid form, for example in the form of a powder, coated in the inside of the canula **3** (see Fig.2A) or in the outside of the canula **3** (see Fig.2B). The first and the second composition may be contacted and mixed in the canula, when the operator pushes the plunger and triggers the passage of the first composition through the internally coated canula.

In a third embodiment (see Fig.2C), the second composition **6** is inside the canula **3** but not coated on the walls of the canula **3.** In this embodiment, the first and the second composition may also be contacted and mixed in the canula **3,** when the operator pushes the plunger and triggers the passage of the first composition through the internally coated canula **3;** in this embodiment, the second composition **6** may be in a liquid or in a solid form.

The device of the invention may comprise at least one filter **5,** preferably a back filter **5a** or a front filter **5b,** or both. The filter **5** may have several functions, among which the prevention of any undesirable movement of the second composition **6** located in the canula.

In another embodiment (not represented), the second composition **6** is in a solid form and is coated both in the inside of the canula **3,** and in the outside of the canula **3.**

According to one embodiment, the second composition **6** may be a pH-adjuster, and the first composition may be a composition not having a physiological pH. The second composition **6** is such that it immediately dissolves when put into contact with the first composition released from the carpule or the cylindrical barrel of the syringe.

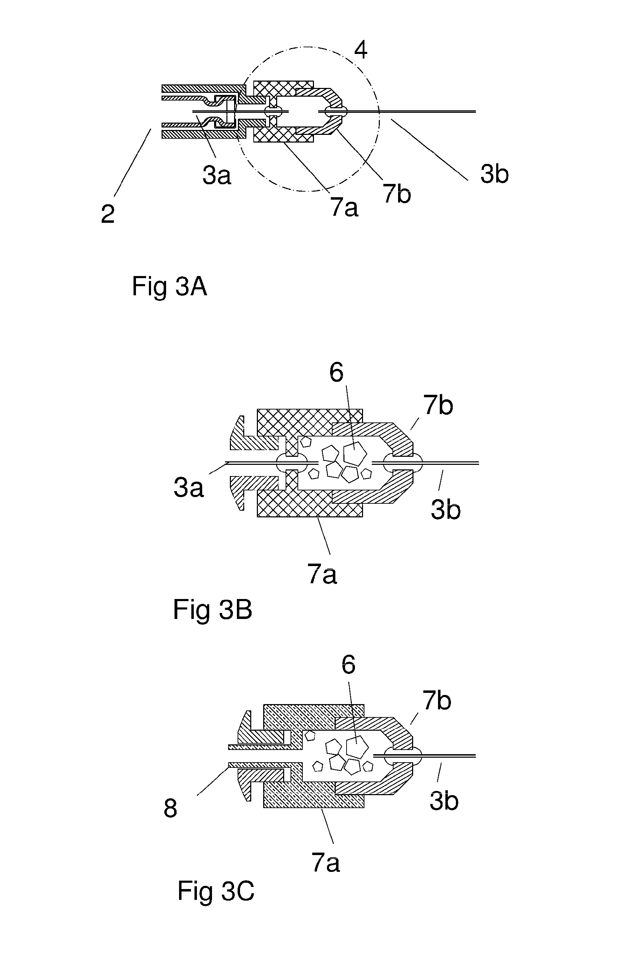

Figures 3A and 3B and 3C show a particular embodiment of the invention wherein the device includes two hubs **7a** and **7b,** a hub **7a** connected to a hub **7b,** so that hub **7a** and hub **7b** define a closed chamber, filled with the second composition **6.** Hub **7a** connects with the needle support member **9** on one end, and to hub **7b** on the other end, and hub **7b** is attached to the canula **3** on the other end.

Figure 3A shows an embodiment of the invention, where the device comprises a specific needle assembly **4** defining a hollow chamber where the second composition **6** is placed and where the mixture occurs.

The first composition is released from the carpule or cylindrical barrel **2** towards the chamber defined by connected hub **7a** and hub **7b** and thus contacted with the second composition **6** in said chamber.

In one embodiment (see Fig. 3A and Fig.3B) the canula **3a** and the canula **3b** may not be connected. In other words, the device may include two distinct canulas. Through the first canula **3a** and upon the action of the operator onto the plunger, the first composition is released into the chamber defined by hubs **7a** and **7b** and contacted with the second composition **6.** Through second canula **3b,** the mixture is expelled out of the device through a further action of the operator on the plunger.

In another embodiment (see Fig. 3C), the first composition is released in the chamber through a corridor **8.**

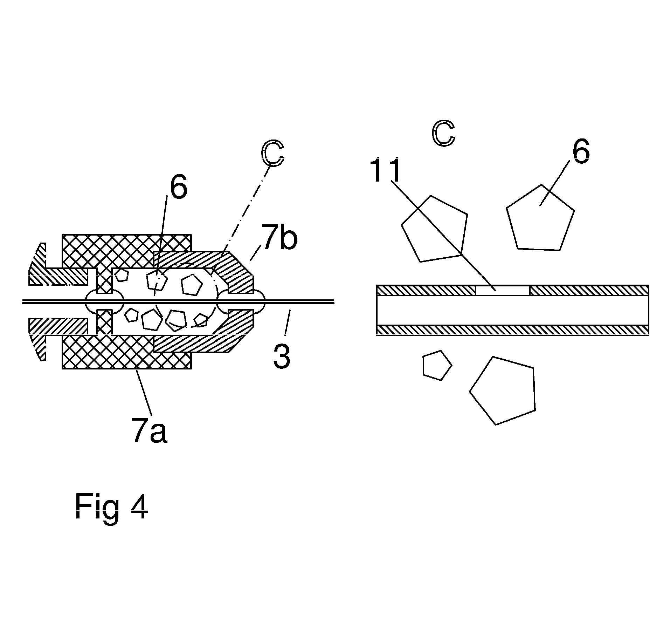

Figure 4 shows a particular embodiment of the invention wherein the device includes a hollow chamber defined by hub **7a** and hub **7b,** as described above in Fig.3, and where the canula **3** has an orifice located within the hollow chamber. This orifice is a lateral opening **11** in the canula, through which first composition and second composition may be put in contact.

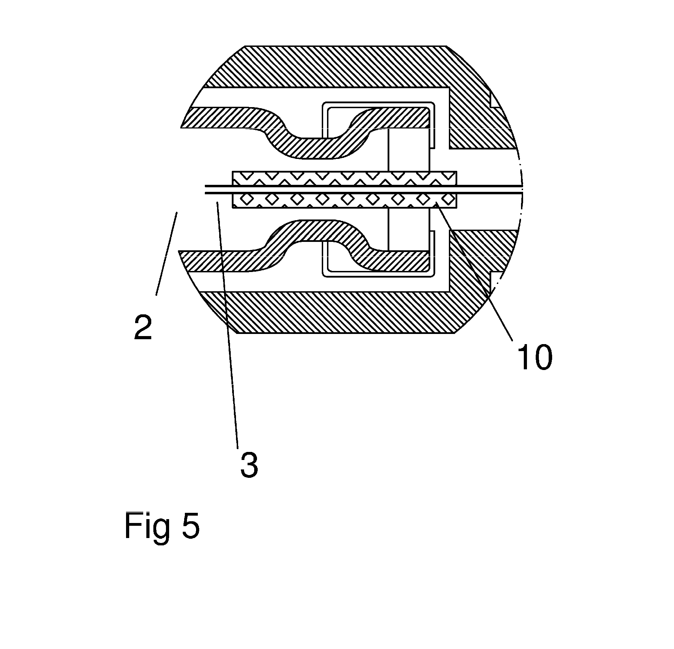

Figure 5 shows an embodiment where the canula **3** may be externally coated with the second composition **6** or may have a container **10** surrounding the canula **3** such that the container **10** contains the second composition **6** and is susceptible to release the second composition **6.** In an embodiment, the part of canula **3** which is externally coated or surrounded by container **10** is the part of the canula **3** which is adapted to be inserted in the carpule or cylindrical barrel **2** containing the first composition. In the embodiment, the release of the second composition **6** may be performed by the mere action of inserting canula **3** in the carpule or cylindrical barrel **2.** In an embodiment container **10** may open or dissolve when contacted with the first composition.

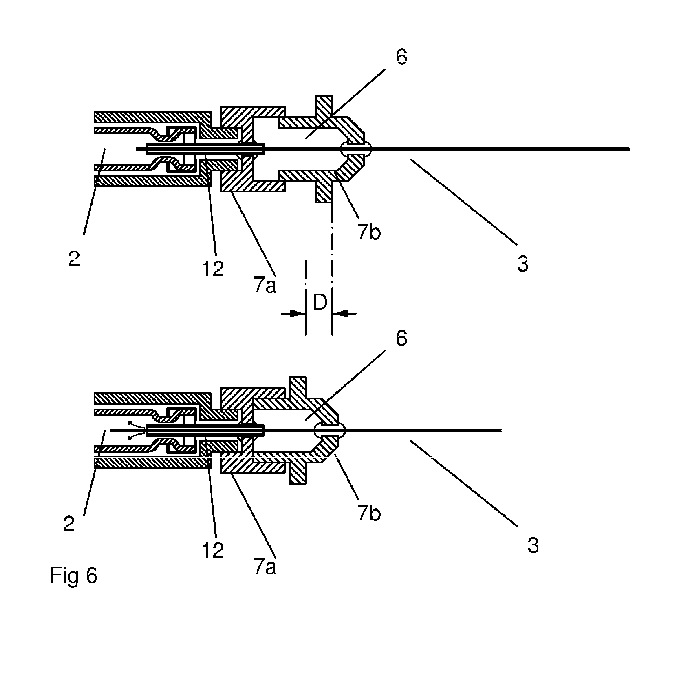

Figure 6 show a particular embodiment of the invention, wherein the device comprises the carpule or cylindrical barrel **2,** a first canula **3,** a second canula **12** surrounding part of canula **3** and two hubs **7a** and **7b** defining a hollow chamber, as already described in the other figures. Canula **3** passes through canula **12.** The hollow chamber is filled with the second composition **6.** In this embodiment, advantageously, the second composition is a liquid. In one embodiment, hub **7b** may be slidably movable in axial direction towards hub **7a.** When hub **7b** is pushed backward to hub **7a,** the volume of the chamber defined by the two hubs decreases, and second composition **6** is pushed into the carpule or cylindrical barrel **2** by means of outer needle **12.** Excess of liquid may released from carpule **2** by means of canula **3.** In this embodiment, the content of the hollow chamber, i.e. the second composition **6,** is brought into the carpule or cylindrical barrel **2** containing the first composition.

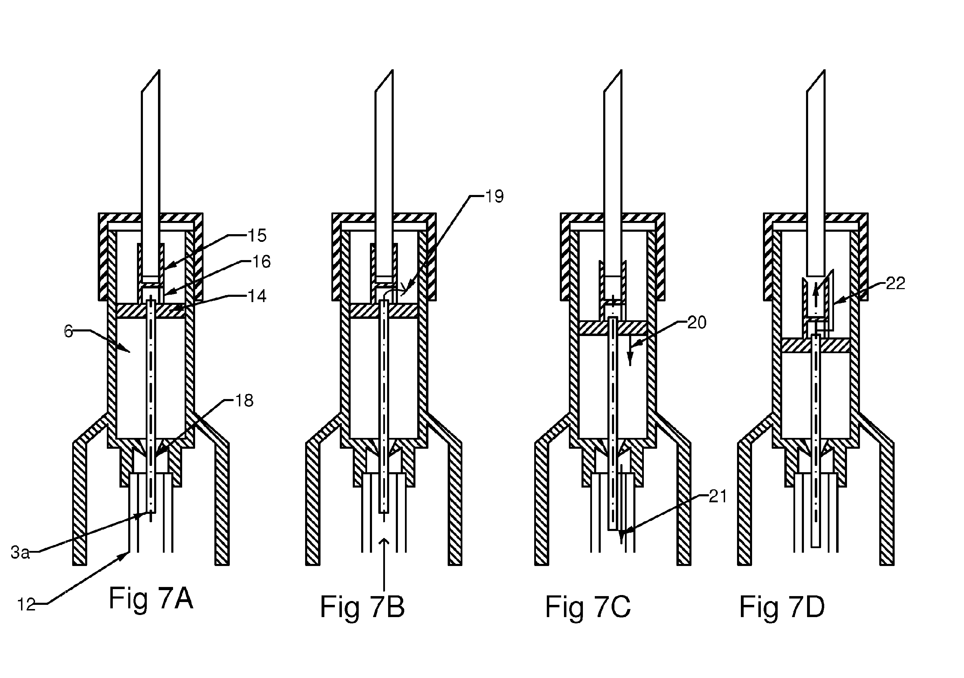

On Figure 7A is shown the device of the invention prior to the mixture; the device comprises an internal canula **3a** and a surrounding canula **12** extending from the hub to the carpule, and an outer canula extending outward for use for injection to the patient ; the hub is separated in two parts by a movable wall **14** having a perpendicular protusion **15.** The protrusion **15** seals the inner end of outer canula. In the walls of protrusion **15** is located an orifice **16.** The outer second composition **6** is encapsulated, preferably in a sterile way, within the lower part of the hub, the upper part of the hub being empty. The encapsulation of second composition was made possible thanks to the presence of a lid **18** located between the lower part of the hub and the canula **12.** The lid **18** is also capable, when a pressure is applied onto said lid 18, to have the second composition pass through to canula **12.** On 7B, is shown that, when the plunger is pushed, the first composition is brought to the upper part of the hub through canula 3a and through hole **16.** When the upper part is being filled, the pressure of the first composition pushes backwards the movable wall **14** and (1) liberates the end of the outer canula from its seal and (2) restricts the volume of the lower part of the hub.

As shown in Figure 7C, the restriction of the lower part of the hub results in applying a pressure on lip **18,** which results in second composition **6** passing through to canula **12** and then to the carpule, where second composition **6** mixes with first composition. In Figure 7C, reference **20** points to a backward arrow intended to show the movement of wall **14.** Reference **21** points to a backward arrow intended to show the movement of second composition **6.**

As shown in Fig. 7D, continuous push on the plunger will then result to reinjecting the mixture in the upper part of the hub through canula **3a** and hole **16,** from where it can be injected through outer canula to the patient.

### [Definitions]

In the meaning of this invention, the following terms have the following meanings:
"injectable composition" means suitable for being introduced in animal, including human, body or its parts, for example but not exclusively by intradermal, hypodermal, intramuscular, intraveinous, or epidural administration ways. It is sterile;
"composition" may be a solid (such as for example a powder), a fluid or a liquid such as for example a solution, a suspension, an emulsion);
"activator" means a second composition having the ability to enhance the properties, such as the efficacy or the biodisponibility of a first composition;
"carpule" means a cartridge;
"cylindrical barrel" means the part of the syringe capable of containing a pharmaceutical composition;
"needle assembly" means a canula and at least one hub surrounding part of the canula and attached to canula;
"needle assembly support member" means the part of the device through which the needle assembly may be connected to the cylindrical barrel or to the carpule;
"pH-adjuster": means an injectable composition capable of lowering or enhancing or buffering the pH of another injectable composition by simple contact between both compositions;
"physiological pH" is a pH ranging from 6.5 to 7.8;
A composition is deemed "not having a physiological pH" when the pH of said composition is below 6.5 or over 7.8;
"prior to injection" means at most one week, preferably at most 48 hours, more preferably at most 12 hours, even more preferably at most 6 hours before injection;
"hub" refers to a piece capable of connecting a canula to the needle member support of a device according to the invention, or more generally to a syringe body;
"canula" refers to a hollow flexible tube, usually containing a trocar at one end, that is inserted into a bodily cavity, duct, tissue or vessel, to administer a substance such as a pharmaceutical composition;
"injection device" means any means of injection comprising a regular syringe, a carpule syringe, a disposable syringe or a reusable syringe;
"plunger" is used for piston or for any piece with a motion similar to that of a piston.

## Claims

1. A method for mixing a first injectable liquid pharmaceutical composition and a second injectable composition within an injection device, said injection device including:
• a generally cylindrical barrel or a generally cylindrical shell in which a carpule is lodged,
• at one first end of the generally cylindrical barrel or the generally cylindrical shell, a plunger, the plunger being mounted on the barrel or shell,
• at a second end opposite the first end of the generally cylindrical barrel or the generally cylindrical shell, is provided a needle assembly support member, on which may be mounted a needle assembly, said needle assembly comprising means to be mounted on the needle support member, and further comprising
• at least one hollow canula for transportation of liquid from the barrel or carpule to the body of the patient,
• and at least one hub surrounding part of the canula and attached to canula; said hub possibly including a hollow part;
**characterized in that** the first injectable composition is within the cylindrical barrel or the carpule and the second injectable composition is associated with the needle assembly, and further **characterized in that** the mixture of said first and said second composition occurs in the hub, in the carpule, or in the canula.

2. A method according to claim **1, characterized in that** the first composition is an injectable composition having a non-physiological pH and the second composition is a buffer of the first composition.

3. A method according to claim **2, characterized in that** the first composition is a dental anesthetic, preferably selected from the group comprising lidocaine, mepivacaine, prilocaine and articaine and mixtures thereof.

4. A method according to claim **2** or claim **3, characterized in that** the buffer has a pK of 6.5 to 7.8, preferably 7 to 7.4.

5. A method according to anyone of claims **2** to **4, characterized in that** the buffer is NaHCO₃.

6. A method according to anyone of claims **1** to **5, characterized in that** the second composition is in the form of a solid, preferably a powder.

7. A method according to anyone of claims **1** to **4, characterized in that** the second composition is in the form of a liquid, preferably a solution or an emulsion.

8. A method according to anyone of claims **1** to **7, characterized in that** the second composition is associated with the needle assembly, preferably the second composition is located on or in the canula, or on or in the hub.

9. A method according to anyone of claims **1** to **7, characterized in that** the mixture is performed prior to injection and occurs in the carpule, in the canula or in the hub.

10. A method according to anyone of claims **1** to **9, characterized in that** the means for having the two compositions be mixed is provoked by a shift of the plunger, a displacement of the carpule or an action on the needle assembly.

11. An injection device (1) including:
• a generally cylindrical barrel or a generally cylindrical shell in which a carpule (2) is lodged,
• at one first end of the generally cylindrical barrel or the generally cylindrical shell, a plunger, the plunger being mounted on the barrel or shell
• at a second end opposite the first end of the generally cylindrical barrel or the generally cylindrical shell, is provided a needle assembly support member (9), on which may be mounted a needle assembly (4), said needle assembly comprising means to be mounted on the needle support member (9), and further comprising
• at least one hollow canula (3) for transportation of liquid from the barrel or carpule (7) to the body of the patient,
• and at least one hub (7) surrounding part of the canula and attached to canula (3); said hub (7) possibly including a hollow part;
**characterized in that** the first injectable composition is within the cylindrical barrel or the carpule and the second injectable composition is associated with the needle assembly, and further **characterized in that** the injection device includes means for mixing the first and the second composition, such that the mixture of said first and said second composition (6) occurs in the hub, in the carpule, or in the canula.

12. An injection device (1) according to claim **11,** wherein the needle assembly comprises a canula (3), and two hubs (7a, 7b), one hub fitting onto the needle support member of the injection device (1), and the other one being attached to the canula (3), both hubs being connected one to another and defining a hollow chamber.

13. An injection device (1) according to claim **11** or claim **12,** includes at least two canulas.

14. An injection device (1) according to anyone of claims **11** to **13,** which is disposable.

15. An injection device (1) according to anyone of claims **11** to **13,** which is reusable.
